# EUROPEAN PATENT APPLICATION

(11) **EP 3 662 884 A1**
(43) Date of publication of application: **10.06.2020**
(21) Application number: 19832263.8
(22) Date of filing: 26.02.2019
(51) Int. Cl.: A61K 8/02, A61K 8/18, A61K 8/33, A61K 8/34, A61Q 19/00, A61Q 19/10

(54) **WET AND REFRESHING WIPE**

(30) Priority: 25.09.2018 ES 201930183 U
(71) Applicant: EURO-GOODNIGHT, S.L., 46880 Bocairent Valencia (ES)
(72) Inventor: LANARAS, Theodoros, 46880 Bocairent (Valencia) (ES)
(74) Representative: Temiño Ceniceros, Ignacio
(86) International application number: PCT/ES2019/070109
(87) International publication number: WO 2020/065105

(57) **Abstract**

The invention relates to a refreshing wet wipe for cleaning the skin and hands of any user, which is preferably offered individually and encapsulated in a plastic container, and preferably intended for airline passengers, and which has the particularity that the composition thereof is constituted by synthetic microfibres compared to conventional cotton wipes, which comprises 75-85% of Polyester and 15-25% of Polyamide; to which the following ingredients are added in a percentage weight ratio therebetween of 99.10-99.50% of water; 0.25-0.30% of phenoxyethanol; 0.18-0.22% of fragrance, which may be white tea or aloe vera; 0.10-0.14% of ethylhexylglycerin; and 0.08-0.12% of benzalkonium chloride.

## Description

### Field of the invention

The present invention consists of a wipe of the type used to refresh and clean hands and which has the particularity that the composition thereof makes it possible to have a high water content, allowing the user to have a great fresh feeling after being used, it is a synthetic but absorbent wipe that replaces the cotton of conventional wipes, and it is a wipe that absorbs water and does not drip.

The invention falls within the textile and cosmetics industry related to the manufacture of refreshing cleaning wipes for the human body, which include chemical products in the composition thereof, and it is especially intended for wipes that are individually dispensed, are wrapped and can be used, for example, in means of transport such as airplanes or trains for passenger comfort and hygiene.

### State of the art

Within the textile industry, different wet wipes intended for cleaning, which are based on a fabric that makes it possible to absorb and clean a specific area of the body, are widely known. Traditionally, Japanese wet wipes, commonly known as *oshibori* towels, are known. They are cotton towels offered at restaurants, which consist of a fabric or towel that is directly moistened with water and is served either on trays or wrapped in plastic.

These types of towels have limited hand and face cleaning power and have evolved into solutions that include different chemical substances that improve the disinfection and cleaning conditions of different types of surfaces.

The subject matter disclosed in documents US5763332 and US6168852, which describe cleaning wipes comprising invert emulsions wherein the continuous phase is oil and the dispersed phase is water, is known, and they additionally comprise different additives that improve their cleaning power.

Wipes having a structure that improves the cleaning of a surface of hands and face, such as those disclosed in document US6183763, which describes a type of wipe with layers that comprise a proton donating agent and which have an acid pH, are also known.

Additionally, the subject matter disclosed in documents US6346506 and EP1146111, which describe wipes impregnated with disinfectant formulations for hard surfaces, or in patent US6258368, which discloses a cleaning wipe comprising absorbent sheets impregnated with antimicrobial compositions, is also known.

Lastly, the subject matter disclosed in document EP1463795, which describes a non-woven fabric cleaning wipe impregnated with a liquid composition comprising disinfecting and pH-adjusting agents, inter alia, is known.

All these solutions are especially intended for cleaning and disinfecting a surface such as that of hands and faces, and have the property that the proportion of chemical components is high, which can affect the sensitivity of a user's hand; therefore, they are not recommendable for wetting and cleaning different parts of the human body.

With respect to direct use in cleaning of body parts, various ways of offering cleaning wipes can currently be found in different restaurants or on board airplanes. On the one hand, we can find duly rolled cotton wipes in a quadrangular receptacle to which hot water is applied before being distributed among the passengers. The problem with this type of product is that the wipe must be moistened before serving it and if those which are not used are kept for several days they must be discarded, since the remaining moisture can cause bacteria harmful to health to proliferate and the product can even rot. Additionally, due to being cotton wipes, during the preparation thereof the fabric must be cut and the ends hemmed so the product does not fray, due to which the wipe can deteriorate relatively easily.

Another option currently used are pre-moistened cotton or non-woven cotton wipes which are hermetically packaged, such as for example those disclosed in document ES0104970U. These types of towels, although they may include perfume-fixing agents, also have the same previously described technical problems of bacteria and hemming, which is that cotton wipes have a composition and structure with cavities that favour fungal and bacterial activity. In this regard, they are only made of viscose and in the form of non-woven fabric, i.e. an imitation handkerchief, whereas the wipe of the present invention is made of polyester microfibres which, while their appearance is similar to that of a cotton wipe, have an improved effect. Additionally, working with polyester makes it possible to imitate forms such as conventional bath towels and other mixed forms and in knitted form or polar fleece, all having an improved absorbent effect with respect to that of usual cotton forms.

Considering the known background, it can be observed that in this industrial sector there is a problem that lies in the need to use an amount of water to moisten the wipes and the problem of finding additives that enable the product to have adequate durability, avoiding the problem of proliferation of bacteria and that this type of product, once used, cannot be recycled to manufacture other types of products. Taking these aspects into account, these technical problems are addressed by means of the present invention, and a wipe is presented with a composition that makes it possible to have lasting solution, that can be wrapped to be individually offered in means of transport or at restaurants, which has no negative implications in its contact with the skin and which, after use, can be recycled for reuse.

### Description of the invention

The present invention describes a refreshing wet wipe that addresses the previously described problems, and which has the particularity that the composition thereof comprises 75-85% of Polyester and 15-25% of Polyamide, to which the following ingredients are added in a percentage weight ratio with respect to total weight of:
- 99.10-99.50% of water;
- 0.25-0.30% of phenoxyethanol;
- 0.18-0.22% of fragrance;
- 0.10-0.14% of ethylhexylglycerin; and
- 0.08-0.12% of benzalkonium chloride.

This composition makes it possible to obtain a wipe with a small percentage of added chemical products, of less than 1%, with respect to a high percentage of water, which allows the wipe to be used in any area of the body and with a very high moistening and refreshing capacity. This refreshing sensation is completed and enhanced by the excellent mild and pleasant smell offered by the fragrance or perfume, which may preferably be white tea or aloe vera.

The process for manufacturing the wipe is as follows. The wipe fabric is manufactured on a continuous basis and is fed rolled to a machine ready for the elaboration, treatment and packaging thereof. The fabric is cut into wipes without need to hem the ends, since this combination of products does not fray. Next, each of the wipes is folded and deposited on a tray. Next, the mixed ingredients are added to the wipe. Lastly, the wipe can be hermetically packaged or packed.

Taking these aspects into account, the wipe described in the present invention has the following advantages, for example, by being an artificial fabric it prevents the growth of bacteria in trapped water. It requires a smaller amount of chemical ingredients to moisten. It has a softer touch, since all the water is removed when wrung, whereas water always remains in cotton wipes, and it can be reused to manufacture other fabrics, such as for example blankets.

Lastly, mention must be made of another two advantages of the present invention, the fact that the cotton-based structure of conventional wipes is replaced, and that it is replaced by a synthetic fabric that absorbs and retains water, thus obtaining individual containers with a durability of five years compared to a durability of one year in the case of cotton. This has a second advantage, which is the possibility of recycling. Specifically, it should be noted that wet cotton used to clean hands or face rots in 10-14 days, which makes recycling unfeasible; whereas the present wipe has a longer duration that enables recycling. Additionally, the non-use of cotton wipes prevents them from entering the food chain when they break and decompose (between 100 and 200 years).

It should be borne in mind that, throughout the description and claims, the term "comprises" and its variants are not intended to exclude other technical features or additional elements. In this regard, with the aim of completing the description and helping to better understand the features of the invention, a detailed description of a preferred embodiment of the present invention is presented below.

### Detailed description of a preferred embodiment of the invention

A preferred use of the invention proposes a refreshing wet wipe to be offered in an airplane, having a composition of 80% of Polyester and 20% of Polyamide, and which additionally comprises the following ingredients with a percentage weight ratio therebetween of:
- 99.30% water;
- 0.28% of phenoxyethanol;
- 0.20% of white tea fragrance;
- 0.12% of ethylhexylglycerin; and
- 0.10% of benzalkonium chloride

With regard to packaging, a wipe weighing 8 grams and with a quadrangular shape 21 cm wide x 21 cm high is proposed, which makes it possible to fold and package it in conventional 17 cm long x 4 cm high plastic containers. These containers are preferably intended for offering the product to airline passengers.

## Claims

1. A refreshing wet wipe for cleaning the skin and hands of any user, **characterized in that** the composition thereof is constituted by synthetic microfibres comprising 75-85% of Polyester and 15-25% of Polyamide; and to which the following ingredients are added with a percentage weight ratio therebetween of:
- 99.10-99.50% of water;
- 0.25-0.30% of phenoxyethanol;
- 0.18-0.22% of fragrance;
- 0.10-0.14% of ethylhexylglycerin; and
- 0.08-0.12% of benzalkonium chloride.

2. The refreshing wet wipe, according to claim 1, **characterised in that** the fragrance is white tea.

3. The refreshing wet wipe, according to claim 1, **characterised in that** the fragrance is aloe vera.
